# EUROPEAN PATENT SPECIFICATION

(11) **EP 2 416 741 B1**
(45) Date of publication and mention of the grant of the patent: **11.03.2015**
(21) Application number: 10711941.4
(22) Date of filing: 06.04.2010
(51) Int. Cl.: A61F 2/46, B01F 11/00, B01F 13/00, B05C 17/005, A61K 9/00

(54) **IN SITU GELLING ALGINATE SYSTEMS**
ALGINATSYSTEME FÜR IN-SITU-GELBILDUNG
SYSTÈMES D'ALGINATE COAGULANT IN SITU

(30) Priority: 06.04.2009 EP 09157419
(43) Date of publication of application: 15.02.2012
(73) Proprietor: Bender Analytical Holding B.V., 6611 KH Overasselt (NL)
(72) Inventor: BENDER, Johannes Caspar Mathias Elizabeth, 6611 KH Overasselt (NL); MANN, Maryssa Gudrun Ailsa, NL-3011 TN Rotterdam (NL); KAMPS, Michael, NL-1442 NZ Purmerend (NL); PELLIKAAN, Hubert, Clemens, 3572CA Utrecht (NL)
(74) Representative: Nederlandsch Octrooibureau
(86) International application number: PCT/NL2010/050174
(87) International publication number: WO 2010/117266

(56) References cited:
- EP-A1- 0 693 292
- DE-A1-102006 029 500
- US-A- 5 618 561
- US-A1- 2005 118 320

## Description

### TECHNICAL FIELD OF THE INVENTION

The invention relates to a system that can suitably be used for the *in situ* formation of biocompatible and biodegradable alginate gels for post operative implantation, sealing and external wound dressing. The alginate-based gelling system of the present invention enables *in situ* formation of a homogeneous, strong and dry (i.e. does not sweat) cross-linked alginate film to be used for soft tissue implantation purposes, slow or prolonged drug release, as a sealant or a wound dressing.

### BACKGROUND OF THE INVENTION

Homogeneous calcium alginate films and foams are not new in the medical device, pharmaceutical and food industries. For many years various manufacturing processes have depended on the fact that aqueous sodium alginate solutions can be cross-linked by multivalent cations, notably Ca²⁺. This is achieved most homogenously when the un-dissolved calcium source, used for cross-linking, is slowly and evenly released in the mixture to be cross-linked. Such a mixture may be mechanically stirred to optimise homogeneity of the subsequently cross-linked gel. Once the calcium ions are released they cross-link the guluronic and mannuronic sites in the sodium alginate.

Johansen and Flink (Biotechnol.Lett., (1985) 7(10):765-8; Biotechnol.Lett., (1986), 8(2):121-6; Enzyme Microbial Technol., (1986a), 8(3):145-8; Enzyme Microbial Technol., (1986b) 8(12):737-48) developed techniques for the immobilization of yeast cells. The authors apply internal gelation principles, which were originally developed for food gels. Briefly, an aqueous dispersion of sodium alginate, an insoluble calcium salt and D-glucono-1,5-lactone is created; the lactone gradually hydrolyses forming gluconic acid. The formation of acid lowers the pH and in turn releases calcium ions. Gelation occurs progressively from within the solution. This gelation technique is also described in patent US 6,497,902 (The Regents of the University of Michigan) which discloses a method for the preparation of ionically cross-linked hydrogels with adjustable gelation time.

Furthermore, WO 2007/103208 A2 (FMC Biopolymer, FMC Corporation) describes a similar method for the creation of gelled biopolymer based foams employing ionic gelling agents and pH modifiers. The foams are prefabricated by blending the foam forming components with a dynamic mixer. The foaming agent may be a cellulose derivative or a non-ionic surfactant. A polyol is present in a ratio not more than 10 times that of the gel forming polymer and is utilized exclusively as a plasticizer. The process is completed by a drying step, which affords soft, pliable, highly absorbent foams for use, in some instances, as wound dressing material or barriers preventing tissue adhesion. The homogeneous foams described in WO 2007/103208 are not formed *in situ.*

CA 2,203,367 (Gilchrist Ltd.) describes a foamable formulation and a foam comprising such substances as alginate, carboxymethylcellulose, collagen and others, with foaming agents cetrimide, lecithin, silicone or surfactants. Said foam may be dispensed from a closed container comprising two separate reservoirs. Optionally one of the reservoirs may contain an active ingredient which is intended to be mixed throughout the composition of the second reservoir upon application. The components of the formulation may be extruded from the reservoirs and mixed or sprayed by means of an aerosol. The methodology described in CA 2,203,367 does not create a homogeneously cross-linked gel or fleece *in situ.*

US 3,640,741 (Hollister Incorporated) describes a plastic, gel containing composition which is a slowly water soluble device for insertion into the body. The device is prepared by cross-linking a hydrophilic colloid, of which alginate gum may be used, with propylene glycol in a organic water-soluble liquid medium such as glycerol. The US patent describes a moulding step that comprises extruding the reaction mass while it is gelling to plastic consistency, the method of extrusion however, is not described. Illustrations portray a plastic ring-shaped mould in which the plastic composition is formed. The prefabricated gel compositions described in US 3,640,741 are intended for use as a vehicle for delivering medication. These compositions are said to have excellent pressure sensitive adhesive properties.

US 2005/0118320 (J. Manheimer, Inc.) describes a method and composition for a gel mixture whereby an alginate gel, intended for use in the food industry, is created by the mixing of two separate components. The first is a liquid mixture comprising alginate blended with sugar and solubilised in water and ethyl alcohol, the second component is a powder blend of sugar, calcium, acids (adipic and glucono delta lactone) and sodium citrate. The method described in US 2005/0118320 utilizes a two component system which is mixed by shaking and is allowed to set for up to 30 minutes. Gel systems that gel at such a slow rate are not suitable for *in situ* use during surgery.

US 6,150,581 (United States Surgical Corporation) describes a method for preventing post surgical adhesions by the *in situ* creation of a chitosan / alginate dressing. A solution containing chitosan and a complexing agent and a solution containing alginate are combined by spraying each fluid onto a target location at the site of surgical intervention. More specifically a method is described wherein the chitosan / complexing agent solution and the alginate solution are sprayed simultaneously by separate sprayers. Chitosan / alginate gels such as those described in US 6,150,581 are predominantly cross-linked by a direct interaction between the two polymers which form a polyelectrolyte complex. These gels are often brittle and lack homogeneity

US 5,266,326 (Pfizer Hospital Products Group, Inc.) describes a method of modifying salts of alginic acid *in situ* for preventing and treating various post-operative intra-articular and extra-articular complications. The method comprises injecting an alginate solution followed by a cross-linking solution of water soluble salts for the *in situ* formation of an insoluble gel, or simultaneously injecting an alginate solution and a cross-linking solution of water soluble salts through a double lumen needle. The cross-linking solution is a solution of water soluble cations. Although the gel would be formed *in situ,* homogeneity would be near impossible to achieve using this sequential gel-forming approach.

US 2007/0179117 A1 (Sughrue Mion, Pllc) discloses injectable cross-linked and uncross-linked alginates for use in cosmetic surgery, for the treatment of gastrooesophageal reflux, urinary incontinence and vesico-ureteral reflux. Implantable microcapsules, micro-particles or gels are produced from alginates. Alginates are cross-linked with polyvalent cations, or are uncross-linked; they are intended for the correction of folds and wrinkles and increasing tissue volume. The alginate solution, containing glucono delta lactone is injected into the target location followed by the component comprising calcium or barium salts and EDTA or citrate solution. Unlike the gelling compositions disclosed in US 5,266,326, this formulation includes gelation rate controllers and sequestrants, but the sequential injection and lack of proper mixing would likely result in a lack of homogeneity.

US 6,022,556 (Johnson & Johnson Medical, Inc.) discloses water swellable wound dressing material comprising a mono- or polyhydric alcohol, an alginate ester of a polyhydric alcohol, a humectant (here interpreted as polyol) and water. Gelation is achieved by ionically cross-linking the non-esterified alginate groups of the alginate ester with polyvalent cations. A method is provided whereby the gelled dressing is prefabricated using a sequential process comprising mixing, spreading and drying. Heating the dressings to 80° C dries the material thoroughly before it is packaged or used.

US 6,638,917 and US 6,693,089 (Boston Scientific SciMed, Inc) describe a method of reducing adhesion at a site of trauma by the formation of ionically cross-linked sodium alginate anti-adhesion barriers. The method includes forming a film from an alginate solution, optionally on a substrate. The film is contacted with a cross-linking solution to form a cross-linked mechanically stable sheet having a thickness in a range of 0.25 mm to 10 mm. At least a portion of the prefabricated sheet is placed at the site of trauma. The sheet has a tear strength sufficient for suturing and repositioning. The US patents also mention that a cross-linked alginate film may be formed *in situ* by placing a prefabricated non-cross-linked film at the site of trauma after which the cross-linking solution is applied to the film or *vice versa*. The US patents further teach to use glycerol and sorbitol in the storage of the film.

WO 2006/044342 A2 (FMC Biopolymer AS) describes self-gelling alginate compositions and kits as well as methods of making and using these compositions. The gelling composition taught by this international patent application comprises a two component system in which the first is a solution of soluble alkaline earth salts of alginic acid; in which the second is an insoluble alginate/gelling ion dispersion. The two fluids are extruded and mixed through a T-connector, upon extrusion, the mixture undergoes spontaneous, but relatively slow gelation. Unlike the aforementioned prior art references, WO 2006/044342 describes *in situ* self-gelling compositions designed to be dispensed both into the individual as well as onto an external skin surface or wound.

DE 10 2006 029 500 A1 describes the use of a multi-component agent comprising two additives which hardens to an alginate mass. The first additive comprises a non-aqueous alginate solution and a calcium salt using glycerin/polyalkyleneglycol as solvent. The second additive comprises water, sodium polyacrylate and diatomaceous earth.

US 2005/118320 A1 describes a gel mixture prepared from a liquid mixture and a powder blend. The liquid mixture is an alginate blended with sugar and solubilized in water and optionally ethanol. The powder blend is a mix of sugar, acids, calciumglycerophosphate and sodium citrate. The liquid mixture and powder blend are combined to form a gel.

US 5618561 describes a process for preparing a gel, said process comprising providing an aqueous earth alkaline alginate, a polyhydric alcohol and a polymer having a hydrophilic character selected from the group consisting of hyaluronic acid and a derivative thereof and, subsequently extruding the fluid gel obtained by pumping it through a slit of adjustable width and thickness to provide an extruded film and coagulating said extruded film by passing it through from 2 to 4 successive coagulation baths.

EP 0693292 A1 describes aqueous gel compositions for use as wound dressings, comprising from 2% to 10% w/v of alginate and from 25% to 40% w/v of polyhydric alcohol, and further describes a process of making such gel compositions comprising the steps of providing an aqueous gel having the above composition and sterilising the aqueous gel.

Several of the gel-forming systems described herein before are not suitable for surgical use and of those that are suitable, most cannot be used for *in situ* formation of an alginate gel, i.e. the gel must be prepared prior to implantation. In addition, the gelling systems from the prior art that are capable of *in situ* gel formation suffer from the drawback that they do not form homogeneous gels meaning that these *in situ* formed gels are not uniform with regard to strength, flexibility and transparency. Lack of uniformity will result in uneven release of active ingredients, unpredictable biodegradation and when the gel is used for sealant purposes, weak points in the gel may result in leakage. Consequently, there is an as yet unmet need for gelling systems that can be introduced into the body during surgery and / or onto the body after e.g. injury, and that will form a homogeneous alginate gel *in situ.*

### SUMMARY OF THE INVENTION

The inventors have developed a system that can be used to introduce a gel-forming fluid into or onto the body that will produce a homogeneous gel *in situ.* The system according to the present invention comprises:
- a first reservoir comprising a first flowable composition in the form of an aqueous solution of ionically cross-linkable alginate;
- a second reservoir comprising a second flowable composition, said second flowable composition containing one or more polyols;
- a first outlet from the first reservoir;
- a second outlet from the second reservoir,
- said first outlet and said second outlet being arranged in such a way that the first flowable composition is mixed with the second flowable composition when both compositions are simultaneously expelled from the respective reservoirs, thereby forming the gel-forming fluid;
wherein the first and / or second flowable composition contains a cross-linking agent and wherein upon admixture of the first flowable composition with the second flowable composition said cross-linking agent initiates the formation of a solid or semi-solid ionically cross-linked alginate gel.

The inventors have unexpectedly discovered that the presence of a substantial amount of polyol in the second flowable composition favourably affects the cross-linking reaction between the cross-linking agent and the alginate. More particularly, it was found that the polyol acts as a temporary sequestrant of the multivalent cation delaying gelation until after the mixed composition has been expelled from the device and providing for a more homogeneously cross-linked gel. The gels produced by the present system are homogeneous, dry (i.e. do not sweat), softer, stronger and more flexible when water is partly or wholly replaced by a polyol.

The present invention encompasses a delivery system in which the second flowable composition contains an active cross-linking agent that will immediately react with alginate contained in the first flowable composition when the first and second flowable composition are mixed. Alternatively, the first or second flowable composition may contain an inactive cross-linking agent that is activated under the influence of an activator. A suitable example of a combination of an inactive cross-linking agent and an activator is the combination of an undissolved calcium salt and an acid that, following admixture of the flowable compositions, will render the calcium salt largely soluble. The use of an inactive cross-linking agent in combination with an activator offers the advantage that the rate of gelation can be controlled very effectively. Depending on the actual purpose for which the present system is used, it may be desirable to either produce a gel-forming fluid that gels very quickly, or to form a fluid that gels relatively slowly. The present system is further suitable for producing adhesive gels that can be used, for instance, as sealants or for producing gels that are non-adhesive and that can be used, for instance, to prevent post-surgical tissue adhesion.

The system according to the present invention can advantageously be employed for a variety of medical or veterinary uses. Examples of such uses include:
- repair of physical damage to living tissues such as breaks, cuts and tears caused by unintentional injury.
- post operative repair of living tissue after injury resulting from surgical procedures.
- repair and sealing of membranous biological tissues, including the dura mater.
- forming of a homogeneous hydrogel that seals biological tissues creating a water tight seal within a desirable time period.
- forming of a water tight seal to prevent the leakage of biological fluids, such as cerebrospinal fluid.
- application in biological spaces where minimal swelling of the gel is advantageous.
- application in biological spaces as a delivery vehicle of viable cells.
- application as a delivery vehicle of a pharmaceutical or therapeutic agent.

### BRIEF DESCRIPTION OF THE DRAWINGS

FIG. 1 is a perspective view of a system for delivery of a gel-forming fluid according to the present invention.
FIG. 2 is a top view of the delivery system illustrated in FIG. 1.
FIG. 3 is a cross-sectional view of the delivery system illustrated in FIG. 1
FIG. 4 shows how dexamethasone was released over time from an alginate gel that was produced with a delivery system according to the present invention
FIG. 5 shows how dexamethasone was released from an alginate gel containing dexamethasone loaded microparticles of a lipid (Compritol® 888)
FIG. 6 shows how insulin and albumin were released over time from an alginate gel that was produced with a delivery system according to the present invention

### DETAILED DESCRIPTION OF THE INVENTION

Accordingly, one aspect of the present invention relates to a system for delivering a gel-forming fluid, comprising:
- a first reservoir comprising a first flowable composition in the form of an aqueous solution of ionically cross-linkable alginate, said solution containing 0.3-7 wt.% of the alginate;
- a second reservoir comprising a second flowable composition, said second flowable composition containing 30-100 wt.% of one or more polyols; and optionally up to 50 wt.% of water;
- a first outlet from the first reservoir;
- a second outlet from the second reservoir,
- said first outlet and said second outlet being arranged in such a way that the first flowable composition is mixed with the second flowable composition when both compositions are simultaneously expelled from the respective reservoirs, thereby forming the gel-forming fluid;
wherein (i) the first and/or second flowable composition contains 0.1-5 wt.% of an inactive cross-linking agent and the second flowable composition contains 0.01-5 wt.% of an activator or (ii) the second flowable composition contains 0.5-50 wt.% of an active cross-linking agent; and wherein upon admixture of the first flowable composition with the second flowable composition the cross-linking agent initiates the formation of a solid or semi-solid ionically cross-linked alginate gel.

The term "flowable" as used herein refers to ability of a non-gaseous material to continually deform (flow) under an applied shear stress. Typical examples of a flowable material include liquids, visco-elastic fluids, pastes and free flowing powders. Preferably, the first flowable composition is a liquid or a visco-elastic fluid. Most preferably, the first flowable composition is a liquid. Likewise, also the second flowable composition preferably is a liquid or a visco-elastic fluid. Most preferably, the second flowable composition is a liquid.

The present invention provides a system for the creation of a homogeneously cross-linked alginate gel for use in numerous biomedical applications. The present system is capable of providing for diverse gelling times and gel strengths in line with the intended purpose; providing for instant or gradual gelation as required. The primary embodiments of the present system require no complex formulation techniques and avoid the formation of discrete gelling particles that may be subject to flocculation and sedimentation.

An important element of the present system resides in the fact that the outlets of the first and second reservoirs are arranged in such a way that the first flowable composition is mixed with the second flowable composition when both compositions are simultaneously expelled from their respective reservoirs. Here the term "expel" means that the flowable compositions are driven out of the respective reservoirs, e.g. because a user pushes a syringe piston. In accordance with one embodiment of the invention, the first outlet and the second outlet may take the shape of a first nozzle and a second nozzle. By positioning the first nozzle and second nozzle closely together and by pointing the nozzles in the same direction intimate mixing of the first flowable composition and the second flowable composition can be achieved. According to another embodiment, the first outlet and the second outlet connect the first reservoir and the second reservoir to a mixing chamber in which the first flowable composition is thoroughly mixed with the second flowable composition after which it is expelled from the mixing chamber, e.g. through an aperture, a needle or a nozzle.

In accordance with a particular preferred embodiment of the present invention, the system comprises a syringe, said syringe comprising a static mixing unit that is connected to the first outlet of the first reservoir and the second outlet of the second reservoir and which static mixing unit comprises an outlet for dispensing the gel-forming fluid resulting from the admixture of the first flowable composition and the second flowable composition. In such a dual reservoir syringe, when the plunger is pressed, the first and second flowable composition are introduced into the static mixer in which both flowable compositions are homogeneously mixed prior to application of the resulting gel-forming fluid onto a surface or into a cavity.

The present system comprises at least two different flowable compositions: one comprises an aqueous alginate solution; the other contains a considerable amount of one or more polyols. When the two components are combined, an alginate gel begins to form as the cross-linking agent starts to react with the alginate component. The rate and extent of gelation are dependent on several factors including: the volume ratios in which the two flowable compositions are combined; the concentration of soluble alginate; the molecular weight of the alginate; the concentration of the cross-linking agent; and the polyol concentration. In case an inactive cross-linking agent is used, the concentration of activator also affects the rate and extent of gelation. As explained herein before, when the present system is used to deliver the gel-forming fluid, said gel-forming fluid is formed by the admixture of the first and second flowable composition. The gel-forming fluid can be dispensed from the present system to a location on or within the body where it will form a gelled alginate device *in situ.*

The one or more polyols employed in the present system are advantageously selected from the group consisting of glycerol, sorbitol, mannitol, xylitol and combinations thereof. Preferably, the one or more polyols are selected from sorbitol, glycerol and combinations thereof.

The present system is designed for biomedical uses. In order to reduce the risk that such uses may cause infections it is preferred that both the first flowable composition and the second flowable composition are sterile.

Crude alginates naturally contain high levels of endotoxins. It will be readily understood that for biomedical uses the endotoxin levels of the alginate must have been reduced to safe levels. Accordingly, in a preferred embodiment the first flowable composition contains 0-175 Endotoxin Units (EU). For the present system the total number of endotoxins units preferably does not exceed 350. Accordingly, in a preferred embodiment each flowable composition is required to contain not more than 175 EU irrespective of size or volume. The present system is particularly suitable for use in cranial and/or spinal areas. For these types of applications it is desirable that the flowable compositions together contain less than 15 endotoxin units. Likewise, the alginate employed in the system advantageously contains less than 50 EU, more preferably less than 30 EU per gram.

In a preferred embodiment the alginate is ultrapure sodium alginate with a high G-block to M-block ratio, e.g. a G-block to M-block ratio of at least 60:40, although sodium alginate with a range of G-block to M-block ratios may be used, including high M-block to G-block.

The ionically cross-linkable alginate of the present invention preferably is an alkaline earth metal alginate, e.g. sodium alginate or potassium alginate.

High molecular weight alginates have proportionately higher viscosities which allow for better support of the cross-linking agent in suspension, lessening the degree of flocculation and sedimentation. Consequently, according to another preferred embodiment, the alginate employed in the present system is high molecular weight alginate having a molecular weight of at least 500,000 Da. The molecular weight and viscosity of the alginate determines the weight percentage of the cation source required for cross linking; higher molecular weight alginates require fewer cations for cross-linking. Thus, in case calcium carbonate is used as an inactive cross-linking agent in combination with an acid activator, release of carbon dioxide can be controlled by selecting an alginate having the appropriate molecular weight. By manipulating the amount of carbon dioxide that is released during the gelation process the strength of the resulting alginate gel can be controlled.

The present system advantageously contains an alginate that is capable of a high viscosity at relatively low concentrations. Preferably, the first flowable composition contains an alginate that, when added to distilled water in a concentration of 1 wt.%, is capable of inducing a viscosity of at least 180 mPa.s at a temperature of 20 °C, said viscosity being determined using a Brookfield DV viscometer (TF spindle) at a shear rate of 1 s⁻¹.

The use of an inactive cross-linking agent in the present system offers the advantage that the rate of alginate gelation can be controlled effectively, depending on the rate of activation of the inactive cross-linking agent. According to a very preferred embodiment, the inactive cross-linking agent is a water-insoluble salt of a multivalent cation. Examples of multivalent cations include multivalent metal cations as well as multivalent cationic substances, such as gentamicin or cationic peptides. It should be understood that the valency of cationic substances can be dependent on the pH of the flowable composition in which it is contained. Even more preferably, the inactive cross-linking agent is a water-insoluble salt of a multivalent metal cation selected from the group consisting of Ca²⁺, Mg²⁺, Zn²⁺, Ba²⁺ and combinations thereof. Most preferably, the inactive cross-linking agent is a water-insoluble salt of Ca²⁺. Here the term water-insoluble salt refers to a salt having a solubility in distilled water at 20 °C of less than 0.001 mol/l, preferably less than 0.0005 mol/l, more preferably of less than 0.0003 mol/l, most preferably of less than 0.0002 mol/l.

The inactive cross-linking agent is advantageously homogeneously dispersed throughout the first flowable composition. The alginate contained in the same first flowable composition renders said composition viscous. This viscosity, when high, is advantageous as it will prevent dispersed components, e.g. dispersed inactive cross-linking agent, from settling, i.e. from forming a sediment. Accordingly, in accordance with a preferred embodiment of the invention, the first flowable composition has a viscosity of at least 180 mPa.s at a temperature of 20 °C, using a Brookfield DV viscometer (TF spindle) at a shear rate of 1 s⁻¹.

In case the alginate contained in the same first flowable composition does not render said composition highly viscous, inactive cross-linking agent is preferably contained within the same composition in the form of nanoparticles, having a weight averaged diameter within the range of 50-300 nm. Particles having such a small diameter offer the advantage that they will remain in suspension for a prolonged period of time, especially if such a suspension is highly viscous.

A first aspect of the present invention relates to a system wherein the first flowable composition contains 0.1-5 wt.% of the inactive cross-linking agent and has a pH in the range of 7.0-10.5 and the second flowable composition contains 0.01-5 wt.% of the activator and has pH in the range of 1.5-4. When this system is used to produce a gel-forming fluid, the first flowable composition containing the alginate and the inactive cross-linking agent is combined with the second flowable composition containing the activator. Under the influence of the activator the inactive cross-linking agent will be activated and cross-linking of the alginate will commence. The rate of gelation of the gel-forming fluid is dependent on the rate at which the inactive cross-linker is activated. Thus, this particular system offers the advantage that gelation rates can be reduced to such a level that premature gelation is avoided.

In accordance with a preferred embodiment of the above defined first aspect of the invention, the polyol is selected from sorbitol, glycerol and combinations thereof. Most preferably, the polyol is sorbitol. The inactive cross-linking agent employed in the system according to the first aspect of the invention preferably is calcium carbonate (CaCO₃), more preferably nanoparticulate calcium carbonate. In aqueous environment CaCO₃ can react with an organic acid, thereby releasing Ca²⁺ but also forming CO₂ gas. CO₂ release results in an increase in rigidity and fragility of the alginate gel, therefore a decrease in the weight percentage of CaCO₃ results in less CO₂ gas which translates to a stronger and more flexible gel.

In order to attain similar viscosities for both compositions, it may be necessary to increase the viscosity of the polyol with dissolved or suspended excipients such as calcium phosphate, polymers, hyaluronic acid and nano-hydroxylapatites.

In accordance with another preferred embodiment of the above defined first aspect of the present invention, the solubility of the inactive cross-linking agent in the mixture of the first flowable composition and the second flowable composition is at least 10 times as high, preferably at least 30 times as high as its solubility in the first flowable composition. In case the present system employs a slow acting activator, e.g. an acid precursor such as glucono delta lactone, the aforementioned solubility is to be determined after the activator has become fully active. If the inactive cross-linking agent is a salt of a multivalent metal cation that is essentially insoluble in the first flowable composition and said salt is much more soluble in the mixture of the first and second flowable composition, admixture of these compositions will cause the release of the multivalent metal cations into the gel-forming fluid. These released multivalent metal cations will react almost instantaneously with the alginate component to form an ionically cross-linked alginate gel.

A second aspect of the invention concerns a system wherein the second flowable composition contains 0.1-5 wt.% of the inactive cross-linking agent and 0.01-5 wt.% of the activator, said second flowable composition having a pH in the range of 1.5-7.

In accordance with a preferred embodiment of the second aspect of the invention the polyol is selected from sorbitol, glycerol and combinations thereof. Most preferably, the polyol is glycerol. The inactive cross-linking agent employed in the system according to the second aspect of the invention preferably is gentamicin alginate. Gentamicin may be complexed with alginate to form small beads. These beads can be suitably employed as an inactive cross-linker in the second flowable composition of the system according to the previously defined second aspect.

A third aspect of the invention relates to a system wherein the second flowable composition contains 0.5-50 wt.% of an active cross-linking agent, said second flowable composition having a pH in the range of 4.0-9.0, preferably of 6.5-7.5.

The polyol employed in accordance with the third aspect of the invention is preferably selected from sorbitol, glycerol and combinations thereof. Most preferably, the polyol is glycerol. An example of an active cross-linking agent that may be employed in accordance with this aspect of the invention is calcium phosphate (used in calcium phosphate cements as injectable bone substitute).

The composition of the first of the dual reservoirs of the present system may also comprise, in addition to the alginate, a second polymer. Preferably, the first flowable composition, in addition to the ionically cross-linkable alginate, contains 0.5-10 wt.% of a second polymer. The second polymer is advantageously selected from the group consisting of polypeptides and polysaccharides.

According to one preferred embodiment, the second polymer is albumin.

According to another preferred embodiment, the second polymer is a polysaccharide selected from the group chitosan, chitin and hyaluronic acid. According to a particularly preferred embodiment the second polymer is chitosan. In case the second polymer is chitosan, the present system is advantageously designed in such a way that an alginate-chitosan polyelectrolyte complex is formed when the first flowable composition and the second flowable composition are mixed.

Yet another preferred embodiment of the present invention is a system wherein the first flowable composition contains a multifunctional cross-linking agent selected from the group of 3,4-dihydroxyphenylalanine, dopaquinone, L-tyrosine and oxidized phloroglucinol.

In a preferred embodiment the multifunctional cross-linking agent is 3,4-dihydroxyphenylalanine. Preferably, the multifunctional cross-linking agent is incorporated in the first flowable composition in a concentration of 0.1-10 wt.%, more preferably of 0.5-10 wt.%. The incorporation of a multifunctional cross-linking agent offers the advantage that the gel is covalently bonded to reactive groups on biological tissues, for example the free amine groups of collagen, resulting in tissue adhesion.

Yet another preferred embodiment of the present invention is a system wherein the second flowable composition contains a multifunctional cross-linking agent selected from the group of gluteraldehyde, phthaladehyde and oxidised phloroglucinol. In a very preferred embodiment the multifunctional cross-linking agent is glutaraldehyde. Preferably, the multifunctional cross-linking agent is incorporated in the second flowable composition in a concentration of 0.1-10 wt.%, more preferably of 0.5-10 wt.%. The incorporation of a multifunctional cross-linking agent in the second flowable composition offers the advantage that the gel is covalently bonded to reactive groups on biological tissues for example the free amine groups of collagen, resulting in tissue adhesion.

The second flowable composition of the present system may suitably contain a polymer selected from the group comprising: collagen; recombinant collagen; gelatin; recombinant gelatin; casein; RGD collagen binding protein; carboxymethylcellulose; poly methyl vinyl ether co maleic acid; amine-functionalised PEG; polyamidopolyamines. The inclusion of one or more of these polymers offers the advantage that a range of characteristics may be imparted to the gel, including, but not limited to: adhesive; absorbent; swellable; covalently cross-linkable; therapeutic.

In yet another advantageous embodiment, bio-adhesive properties are imparted to the alginate gel by the inclusion of both a second polymer and a multifunctional cross-linking agent. In a preferred embodiment the second polymer is chitosan and the multifunctional cross-linking agent is glutaraldehyde. In another preferred embodiment, the second polymer is chitosan and the multifunctional cross-linking agent is 3,4-dihydroxyphenylalanine.

As explained herein before, the present system may suitably contain an inactive cross-linker in combination with an activator. Most preferably, the activator employed is an acidulant. Even more preferably, the acidulant is contained in the second flowable composition in a concentration such that after admixture of the first flowable composition and the second flowable composition, the pH of the gel-forming composition decreases to level that is at least 2.0, preferably at least 3.0 pH units below the original pH of the first flowable composition. In accordance with the present invention the aforementioned pH decrease is used to trigger the activation of the inactive cross-linking agent, e.g. because the pH decrease causes solubilisation of an undissolved multivalent cation salt.

Examples of acidulants that may suitably be used in the present system include lactic acid, tartaric acid, glucono delta lactone, gluconic acid, malic acid, citric acid and combinations thereof. An advantageous embodiment of the present system utilizes as the acidulant a combination of glucono delta lactone and one or more acids selected from lactic acid, tartaric acid, gluconic acid, malic acid, citric acid and combinations thereof. The combined use of the slow acting acidulant glucono delta lactone and one or more fast acting organic acids offers the advantage that initial gelation occurs relatively quickly, creating a soft gel that will not flow, drip or deform away from the site of trauma. Following admixture of the first and second flowable composition, the glucono delta lactone is gradually hydrolysed to gluconic acid, which acts over a prolonged period to harden and strengthen the gel, allowing cross-linking to occur completely and uniformly.

Besides a polyol, the second flowable composition may suitably contain a substantial amount of water. Preferably, the second flowable composition contains 0-50 wt.% of water, more preferably 0-30 wt.% of water. The one or more polyols and water together preferably constitute the bulk of the second flowable composition. Advantageously, the one or more polyols and water together represent at least 80 wt.%, more preferably at least 90 wt.% of the second flowable composition.

The first flowable composition and the second flowable composition are typically contained in the present system in a weight ratio that is within the range 5:1 to 1:5. According to another preferred embodiment the present system contains 1-100 ml of the first flowable composition and 1-100 ml of the second flowable composition.

The present system may be used to provide sustained release of a pharmaceutical or therapeutic agent, either locally or systemically. Thus, in accordance with a preferred embodiment the first flowable composition and/or the second flowable composition contains a pharmaceutical agent or a therapeutic agent. Even more preferably, the first flowable composition contains a pharmaceutical agent or a therapeutic agent. For specific applications it may be necessary to combine methods known to specialists in the art of sustained release technology with the described alginate system. For instance the use of PLGA microspheres or polyactive microspheres, which can be suspended in hydrogels.

The present system offers the advantage that it can be formulated so as to afford specific release profiles. Typical modifications include, but are not limited to, pore-size; degree of cross-linking; variation of alginate molecular weight; variation of the alginate concentration; combination with other hydrogel forming polymers; combination with lipophylic substances like oils.

In a preferred embodiment, the pharmaceutical agent is selected from the group of anti-inflammatory drugs (steroidal and non-steroidal anti-inflammatory drugs for example dexamethasone) and antimicrobials.

In another preferred embodiment, the therapeutic agent is selected from the group of therapeutic proteins for example albumin or insulin.

In the present system the two flowable compositions are separated to maintain the fluidity of each individual composition.

Before cross-linking can occur, the gel-forming fluid is dispensed from the dispenser, e.g. through a nozzle or needle, and applied onto a surface or into a cavity. The gel-forming fluid flows freely to cover or fill the target site and cross-linking occurs internally. Spraying of the gel-forming fluid is possible with the aid of compressed gas and a suitably modified tip which allows fine dispersion of the completely homogeneous mixture. In order to spray the gel-forming fluid, gelation and setting must be sufficiently delayed that the composition remains fluid long enough for it to be expelled from the system. This requires that the one or more polyols in the second flowable composition act as a temporary sequestering agent of the active cross-linking agent or the active form of the inactive cross-linking agent. When the gelling speed is sufficiently controlled, substantial volumes of gel-forming fluid can be dosed per operation of the system (up to 100 ml per reservoir).

Another aspect of the present invention relates to a method of preparing a gel-forming fluid, said method comprising:
- mixing a first flowable composition as defined herein before with a second flowable composition as defined herein before; and
- allowing the gel-forming fluid so obtained to form a gel.

According to a preferred embodiment of the present method, the first flowable composition and the second flowable composition are admixed in a weight ratio of 1:5 to 5:1. According to another preferred embodiment, 1-100 ml of the first flowable composition is combined with 1-100 ml of the second flowable composition.

The present method includes a method for the treatment of the human or animal body by surgery or therapy. The present method, however, can also suitably be performed as a method that does not involve the treatment of the human or animal body by surgery or therapy. For, instance, the present method may be employed to produce gel structures that can be used for non-medical purposes.

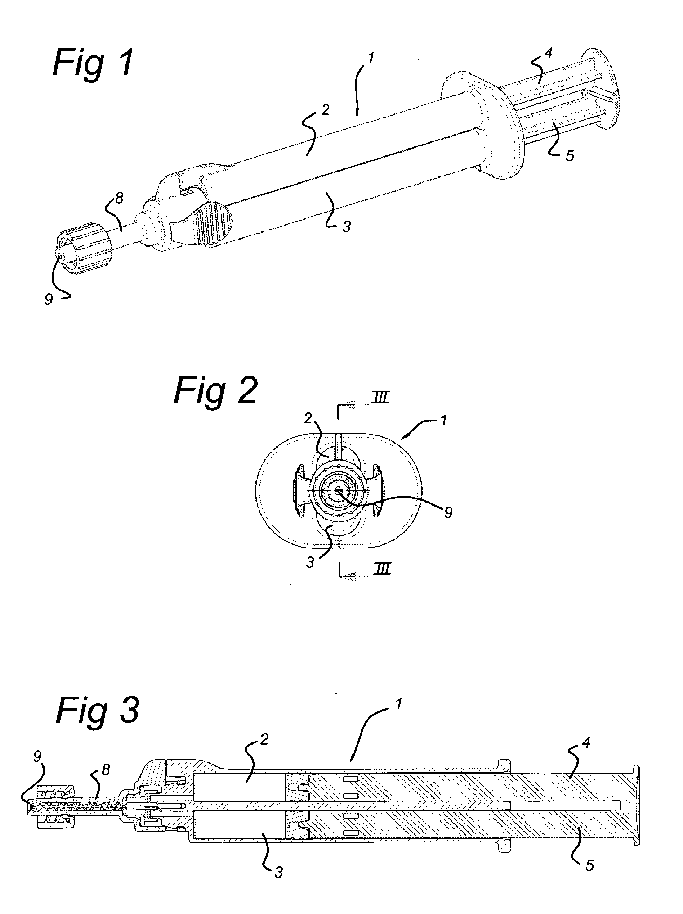

Referring now to FIG. 1, a system for the delivery of a gel-forming fluid is illustrated which allows the first flowable composition and the second flowable composition to become mixed together, before being conveniently delivered to target sites. FIG. 2 shows a top view of the same system. FIG. 3 provides a cross-sectional view of the delivery system, the cross-section being along the line B-B depicted in FIG. 2.

FIG. 3 shows a delivery device **1** comprising a first reservoir **2** that contains the first flowable composition and a second reservoir **3** that contains the second flowable composition. Pistons **4** and **5** can be operated to simultaneously expel the first flowable composition and the second flowable composition from reservoirs **2** and **3**. When expelled from their respective reservoirs, the first flowable composition enters static mixing chamber **8** through outlet **6** and the second flowable composition through outlet **7**. The first and second flowable composition are thoroughly mixed together in mixing chamber **8** before leaving the system as a gel-forming fluid through aperture **9**.

The invention is further illustrated by means of the following examples:

### Example 1

This example describes the dispensing system that can be used to produce an anti-adhesive barrier. The compositions of the first and second flowable composition are depicted in Table 1.

**Table 1**

| **Formulation A** | | | |
|---|---|---|---|
| Reservoir 1 | | Reservoir 2 | |
| Sodium Alginate 3 % w/w ml | 10 | Sorbitol 70 % w/w | 10 ml |
| Nanoparticulate CaCO₃ 0.6 % w/v | | Lactic acid 9 mg/50 µl | 50 µl |
| | | Glucono delta lactone 5 g / 5 ml | 100 mg |

3 g Ultra pure, high molecular weight alginate was weighed into a vessel and a solution was made up to 100 g with deionised water. The suspension was blended with an ultra turrax T25 (Janke & Kunkel, Ika® Labortechnik) after which air bubbles were removed by centrifugation on an Eppendorf 5810 centrifuge at 3000 rpm for 5 minutes. Nanoparticulate calcium carbonate was accurately weighed into a mixing vessel into which the sodium alginate solution was subsequently measured. The calcium carbonate was wetted slightly with de-ionised water prior to the addition of sodium alginate; wetting ensures homogeneous dispersion of the nanoparticles.

Glucono delta lactone was weighed accurately into a vessel containing lactic acid. Sorbitol 70 % w/w was measured into the same vessel and the contents were mixed well. The first reservoir of the syringe was filled with the alginate solution taking care to exclude large air bubbles. The second reservoir of the syringe was filled with the acidic sorbitol solution. Both the static mixer and plunger were then attached and the two solutions extruded through the static mixture which upon setting formed a homogenous clear fleece with an initial setting time of less than 1 minute. Formulations containing glucono delta lactone gradually strengthen over time due to the slow action of the acid.

### Example 2

This example describes dispensing systems that can be used to produce an implant that adheres to biological tissue. The compositions of the first and second flowable compositions employed in these dispensing systems are depicted in Table 2.

**Table 2**

| **Formulation B** | | | |
|---|---|---|---|
| Reservoir 1 | | Reservoir 2 | |
| Sodium Alginate 3 % w/w | 10 ml | Sorbitol 70 % w/w | 10 ml |
| Nanoparticulate CaCO₃ 0.75 % w/v | | Lactic acid 9 mg/50 µl | 75 µl |
| L-DOPA | 300 mg | Glucono delta lactone 5 g / 5 ml | 100 mg |
| | | | |

| **Formulation C** | | | |
|---|---|---|---|
| Reservoir 1 | | Reservoir 2 | |
| Sodium Alginate 3 % w/w | 10 ml | Sorbitol 70 % w/w | 10 ml |
| Nanoparticulate CaCO₃ 0.75 % w/v | | Lactic acid 9 mg/50 µl | 75 µl |
| L-DOPA | 600 mg | Glucono delta lactone 5 g / 5 ml | 100 mg |
| | | | |

| **Formulation D** | | | |
|---|---|---|---|
| Reservoir 1 | | Reservoir 2 | |
| Sodium Alginate 3 % w/w | 10 ml | Sorbitol 70 % w/w | 10 ml |
| Nanoparticulate CaCO₃ 0.75 % w/v | | Lactic acid 9 mg/50 µl | 75 µl |
| L-DOPA | 600 mg | Glucono delta lactone 5 g / 5 ml | 100 mg |
| Chitosan | 200 mg | | |
| | | | |

| **Formulation E** | | | |
|---|---|---|---|
| Reservoir 1 | | Reservoir 2 | |
| (Aq) Sodium Alginate 3 % w/w | 10 ml | Sorbitol 70 % w/w | 10 ml |
| Nanoparticulate CaCO₃ 0.75 % w/v | | Lactic acid 9 mg/50 µl | 75 µl |
| | | Glucono delta lactone 5 g / 5 ml | 100 mg |
| | | Glutaraldehyde 10 % w/v | |

| **Formulation F** | | | |
|---|---|---|---|
| Reservoir 1 | | Reservoir 2 | |
| (Aq) Sodium Alginate 3 % w/w | 10 ml | Sorbitol 70 % w/w | 10 ml |
| Nanoparticulate CaCO₃ 0.75 % w/v | | Lactic acid 9 mg/50 µl | 75 µl |
| Chitosan | 200 mg | Glucono delta lactone 5 g / 5 ml | 100 mg |
| | | Glutaraldehyde 10 % w/v | |

Dual formulations B - F were prepared using the method described in example 1. The burst pressure of each formulation was tested in the following manner. Briefly, bovine dura was dissected into suitably sized sections of tissue. A 3 mm circular incision was cut into each piece of dura, which was then secured to the apparatus in such a manner that simulated body fluid could be forced out under measurable pressure via the circular incision. The formulations were extruded onto the secured dura to form a seal. The pressure of the simulated body fluid was increased to the point that the seal burst; results are depicted in table 3.

**Table 3**

| Formulation | Burst Pressure (mmHg) |
|---|---|
| Formulation A | 3,116 |
| Formulation B | 30.381 |
| Formulation D | 38,95 |
| Formulation E | 38,95 |
| Formulation F | 42,845 |

These results show that the addition of reactive cross-linking agents such as glutaraldehyde significantly increases the adhesion strength of the fleeces to the collagen of the bovine dura.

### Example 3

This example describes the results of a test that was performed to assess the water-absorbing propensity of alginate gels that were produced in accordance with the present invention. The fleeces were prepared as described in example 1 after which their individual masses were recorded. The compositions of the first flowable composition and the second flowable compositions were identical to those of Formulation A (Example 1), except that the additives mentioned in Table 4 were added to either the first or the second flowable composition in the indicated concentration.

**Table 4**

| Sample | Additive | Composition | Concentration (wt.%) |
|---|---|---|---|
| A0 | - | | |
| A1 | DOPA | 1^{st} | 3 wt.% |
| A2 | Carboxy methylcellulose | 2^{nd} | 2 wt.% |
| A3 | Polyvinyl alcohol | 2^{nd} | 4 wt.% |
| A4 | Gelatin | 2^{nd} | 0.2 wt.% |
| A5 | Chitosan | 1^{st} | 2 wt.% |

Fleeces were placed in sealed vessels completely immersed in simulated body fluid over a period of 6 days. At predetermined intervals the fleeces were removed from the fluid, blotted dry and individually weighed. The results so obtained are depicted in Table 5.

**Table 5**

| Day | A0 | A1 | A2 | A3 | A4 | A5 |
|---|---|---|---|---|---|---|
| 0 | 100 | 100 | 100 | 100 | 100 | 100 |
| 1 | 93 | 85 | 135 | 98 | 89 | 111 |
| 2 | 92 | 86 | 134 | 99 | 91 | 111 |
| 3 | 94 | 86 | 136 | 100 | 90 | 111 |
| 5 | 93 | 85 | 135 | 98 | 89 | 111 |

### Example 4

Formulation A was used as an exemplary composition to determine the pH changes and plot a pH profile over the time required for a fleece to set completely. The dual formulation was prepared as described in example 1 however, the mixed components were extruded through the static mixer into a test tube and pH was measured using a Metrohm Porotrobe gel probe and a Metrohm 744 pH meter. Results indicate that the initially opposing pH values of the two compositions are neutralised to approximately pH 6.8 within 5 minutes.

### Example 5

This Example describes the preparation of bone replacement formulations having the compositions depicted in Table 6.

**Table 6**

| **Formulation G** | | | |
|---|---|---|---|
| Reservoir 1 | | Reservoir 2 | |
| Sodium Alginate 3 % w/w | 10 ml | Glycerol | 10 ml |
| | | Calcium Phosphate 1.5 - 2.5 % w/v | |
| | | | |

| **Formulation H** | | | |
|---|---|---|---|
| Reservoir 1 | | Reservoir 2 | |
| Sodium Alginate 3 % w/w | 10 ml | Glycerol | 10 ml |
| Chitosan | 200 mg | Calcium Phosphate 1.5 - 2.5 % w/v | |
| | | Lactic acid 0.0158 % | |

The dual formulations G and H were prepared differently to that of example 1. Briefly, 3 g Ultra pure, high molecular weight alginate was weighed into a vessel and a solution was made up to 100 g with deionised water. The suspension was blended with an ultra turrax T25 (Janke & Kunkel, Ika® Labortechnik) after which air bubbles were removed by centrifugation on an Eppendorf 5810 centrifuge at 3000 rpm for 5 minutes. In the case of dual formulation H, chitosan is weighed accurately, added and stirred into the alginate solution. A single reservoir of the dual syringe was filled with the alginate solution, expressly avoiding the inclusion of large air bubbles.

To glycerol (100 %), calcium phosphate powder and in the case of dual formulation H, additional lactic acid, was added and stirred vigorously. The solution was decanted into the second reservoir of the dual syringe. Both the static mixer and plunger were then attached and the two solutions extruded through the static mixture which upon setting formed a homogenous clear fleece with an initial setting time of less than 1 minute.

This example illustrates how the dual system may be used effectively with calcium sources other than calcium carbonate and polyols such as sorbitol.

### Example 6

Table 7 depicts a formulation that can be employed as an infection care bone implant and that employs no activator.

**Table 7**

| **Formulation 1** | | | |
|---|---|---|---|
| Reservoir 1 | | Reservoir 2 | |
| Sodium Alginate 3 % w/w | 10 ml | Glycerol | 10 ml |
| | | CaPO₄ | 2 g |
| | | Gentamicin alginate | 300 mg |

The gentamicin alginate was prepared as per the methodology in example 10 and the second reservoir composition was prepared as per previously described methods. The fleece produced was white in colour with a homogeneous appearance.

### Example 7

Dispensing systems were prepared in the same way as described in Example 1 on the basis of the formulations depicted in Table 8. The first flowable composition contained 3 wt.% of sodium alginate.

**Table 8**

| Number | First flowable composition Alginate phase | Second flowable composition Polyol phase | Sorbitol 70% | Glycerol 100 % |
|---|---|---|---|---|
| 1 | 0,5 % CaCO₃ | 1% Citric acid | | x |
| 2 | 0,5 % CaCO₃ | 2,2 % hyd. GDL | x | |
| 3 | 2 % CaCO₃ | 2,2 % hyd. GDL | x | |
| 4 | 0,5 % CaCO₃ | 1,65 % hyd. GDL | x | |
| | | 0,045 % lactic acid | | |
| 5 | 0,5 % CaCO₃ | 1,1 % hyd. GDL | x | |
| | | 0,045 % lactic acid | | |
| 6 | 0,75 % CaCO₃ | 1,1 % hyd. GDL | x | |
| | | 0,045 % lactic acid | | |
| 7 | 0,75 % CaCO₃ | 0.5 % hyd. GDL | x | |
| 8 | 0,75 % CaCO₃ | 0,25 % hyd. GDL | x | |
| | | 0,045 % lactic acid | | |
| 9 | 0,75 % CaCO₃ | 0,25 % hyd. GDL | x | |
| | | 0,0225 % lactic acid | | |
| 10 | 0,75 % CaCO₃ | 0,25 % hyd. GDL | x | |
| | | 0,03375 % lactic acid | | |
| 11 | 0,75 % CaCO₃ | 0,5 % hyd. GDL + | x | |
| | | 0,045 % lactic acid | | |
| 12 | 0,75 % CaCO₃ | 0,5 % hyd. GDL + | x | |
| | | 0,03375 % lactic acid | | |
| 13 | 0,75 % CaCO₃ | 0,5 % hyd. GDL | x | |
| | | 0,03375 % lactic acid | | |
| | | 5 % primojel | | |
| 14 | 0,75 % CaCO₃ | 0,5 % hyd. GDL | x | |
| | | 0,03375 % lactic acid | | |
| | | 0.4 % collodion | | |
| 15 | 0,75 % CaCO₃ | 0,5 % hyd. GDL | x | |
| | | 0,045 % lactic acid | | |
| | | 5 % primojel | | |
| 16 | 0,75 % CaCO₃ | 0,5 % hyd. GDL | x | |
| | | 0.045 % lactic acid | | |
| | | 2,5 % primojel | | |
| 17 | 0,75 % CaCO₃ | 0,5 % hyd. GDL | x | |
| | | 0,18 % lactic acid | | |
| 18 | 0,75 % CaC0₃ | 1 % hyd. GDL | x | |
| | | 0,18 % lactic acid | | |
| 19 | 0,75 % CaCO₃ | 0,5 % hyd. GDL | x | |
| | | 0,09 % lactic acid | | |
| | | 10 % primojel | | |
| 20 | 0,75 % CaCO₃ | 0,5 % hyd. GDL | x | |
| | | 0,18 % lactic acid | | |
| | | 10 % primojel | | |
| 21 | 0,75 % CaCO₃ | 0,5h. GDL | x | |
| | | 0,18 % lactic acid | | |
| | | 5 % primojel | | |
| 22 | 0,75 % CaCO₃ | 0,5 % hyd. GDL | x | |
| | | 0,045 % lactic acid | | |
| | | 0,5 % chitosan | | |
| 23 | 0,75 % CaCO₃ | 0,5 % hyd. GDL | x | |
| | | 0,045 % lactic acid | | |
| | | 1 % chitosan | | |
| 24 | 0,75 % CaCO₃ | 0,5 % hyd. GDL | x | |
| | | 0,045 % lactic acid | | |
| | | 2 % chitosan | | |
| 25 | 0,75 % CaCO₃ nano | 0,5 % hyd. GDL | x | |
| | | 0,045 % lactic acid | | |
| 26 | 0,75 % CaCO₃ nano | 0,5 % hyd. GDL | x | |
| | | 0,0225 % lactic acid | | |
| 27 | 0,75 % CaCO₃ nano | 0,5 % hyd. GDL | x | |
| | | 0,009 % lactic acid | | |
| 28 | 0,5 % CaCO₃ nano | 0.5 % hyd. GDL + | x | |
| | | 0,009 % lactic acid | | |
| 29 | 0,6 % CaCO₃ nano | 0.5 % hyd. GDL + | x | |
| | | 0,009 % lactic acid | | |
| 30 | 0,6 % CaCO₃ nano | 0.5 % hyd. GDL | x | |
| | | + | | |
| | | 0,018 % lactic acid | | |
| 31 | 0,6 % CaCO₃ nano | 0.5 % hyd. GDL + | x | |
| | | 0,0135 % lactic acid | | |
| 32 | 0,6 % CaCO₃ coated nano | 0.5 % hyd. GDL + | x | |
| | | 0,009 % lactic acid | | |
| 33 | 0,6 % CaCO₃ nano 1,5 % Chitosan | 0.5 % hyd. GDL + | x | |
| | | 0,009 % lactic acid | | |
| 34 | 0,6 % CaCO₃ nano | 0.5 % hyd. GDL + | x | |
| | 3 % Chitosan | 0,009 % lactic acid | | |
| 35 | 0,6 % CaCO₃ nano | 0.5 % hyd. GDL + | x | |
| | 10 % Transglutaminase | 0,009 % lactic acid | | |
| 36 | 0,6 % CaCO₃ nano | 0.5 % hyd. GDL + | x | |
| | 25 % Transglutaminase | 0,009 % lactic acid | | |

The formulations (Numbers 1-36) were prepared and the dual syringe filled as described in example 1. A number of dual formulations were tested and the results are depicted in Table 9.

**Table 9**

| Nr | Hardening time (min) | Homogeneous | Sweat | Colour | Strength result | Ultimate | Adhesiveness |
|---|---|---|---|---|---|---|---|
| 1 | 0,5 | yes | yes | clear | + | + | None |
| 2 | >10 | yes | no | clear | +++ | ++ | None |
| 3 | 2-3 | yes | no | white | +++ | +++ | None |
| 4 | 2-3 | yes | no | clear | ++ | ++ | None |
| 5 | 1 | yes | no | clear | ++ | ++ | None |
| 6 | 0,5 - 1 | yes | no | clear | ++ | ++ | None |
| 7 | 5 | yes | no | opaque | ++ | | None |
| 8 | 0,5 | yes | no | clear | ++ | +++ | None |
| 9 | 2 | yes | no | clear | ++ | +++ | None |
| 10 | 0,5 - 1 | yes | no | clear | ++ | +++ | None |
| 11 | 0,5 | yes | little | clear | + | ++ | None |
| 12 | 1 | yes | little | clear | + | ++ | None |
| 13 | 3-5 | yes | no | opaque | + | ++ | None |
| 14 | 0,5- 1 | yes | yes | white | + | ++ | None |
| 15 | 0,5 | yes | no | white | + | ++ | None |
| 16 | 1 | yes | no | white | + | ++ | None |
| 17 | 0,3 - 0,5 | yes | yes | clear | +/- | +/- | None |
| 18 | 0,5 | yes | yes | clear | +/- | +/- | None |
| 19 | >10 | yes | no | white | + | +/- | None |
| 20 | 2 | yes | no | white | + | + | None |
| 21 | 0,5 - 1 | yes | little | white | + | + | None |
| 22 | 1-2 | yes | no | orange | + | + | None |
| 23 | 1-2 | yes | no | orange | + | + | None |
| 24 | 1-2 | yes | no | orange | + | + | None |
| 25 | 5-10 sec | no | yes | white | + | ++ | None |
| 26 | 10-20 sec. | no | yes | white | + | ++ | None |
| 27 | 30 sec - 1 min | yes | no | white | + | ++ | None |
| 28 | 30 sec - 1 min | yes | no | clear | + | ++ | None |
| 29 | 30 sec - 1 min | yes | no | opaque | + | ++ | None |
| 30 | 20-30 sec | yes | no | opaque | +/- | +/- | None |
| 31 | 20-30 sec | yes | no | opaque | +/- | +/- | None |
| 32 | 30 sec - 1 min | yes | no | opaque | + | +/- | None |
| 33 | 30 sec - 1 min | yes | no | orange | + | + | None |
| 34 | 30 sec - 1 min | yes | no | orange | + | + | None |
| 35 | 30 sec - 1 min | yes | no | orange | + | + | None |
| 36 | 30 sec - 1 min | yes | no | clear | + | + | None |

The above results demonstrate the robustness of the method as well as the feasibility of such a system for the fabrication of homogeneous gelled alginate films. In addition it is clear from the data that formulations containing the larger particle sized calcium carbonate (Numbers 1-24) require more acid than those containing the nanoparticulate calcium carbonate (Numbers 24 -36). Furthermore, with the exception of very few formulations, the gelling time is relatively fast when compared to prior art containing similar excipients, this may be attributed to the homogeneous mixing of the two components. From the colours observed in fleeces containing compounds such as chitosan or un-dissolved calcium carbonate, it is feasible that the formulations could be stained / dyed with the addition of a non-toxic colouring agent, or they may simply adopt the colour of certain coloured excipients. Sweating may be defined as the contraction of the gel and subsequent squeezing out of water molecules, often attributed to very fast cross-linking speeds. It is desirable for gelation to be controlled so that minimal sweating occurs.

### Example 8

In this example, a dispensing system according to the present invention was used as a vehicle for the delivery of a therapeutic agent. In this instance, the therapeutic agent was dexamethasone. The dual formulation was prepared as per the methodology described in example 1, with the exception of the addition of the dexamethasone. Dexamethasone was weighed accurately into a vessel and the alginate and calcium mixture was added to make up a concentration of 1 mg/ml. After thorough mixing of the components, the dual syringe was filled and assembled as described in previous examples. Release of dexamethasone from the dual formulation after extrusion was measured over time. A fleece "plug", formed from the dual composition, was suspended in simulated body fluid (SBF) and refreshed at 24 hourly intervals. As a simulation of release in a high perfusion location, a flow of 20 ml/24 hours was used. To simulate release in a low perfusion location, a flow of 5 ml/24 hours was used. The concentration of dexamethasone in release medium was measured by way of HPLC analysis using a Dionex Ultimate 3000 HPLC with an Ultimate 3000 variable wavelength detector and a µBondapack C18 (300*3.9* 10 µm) column. Pure dexamethasone was used as the standard.

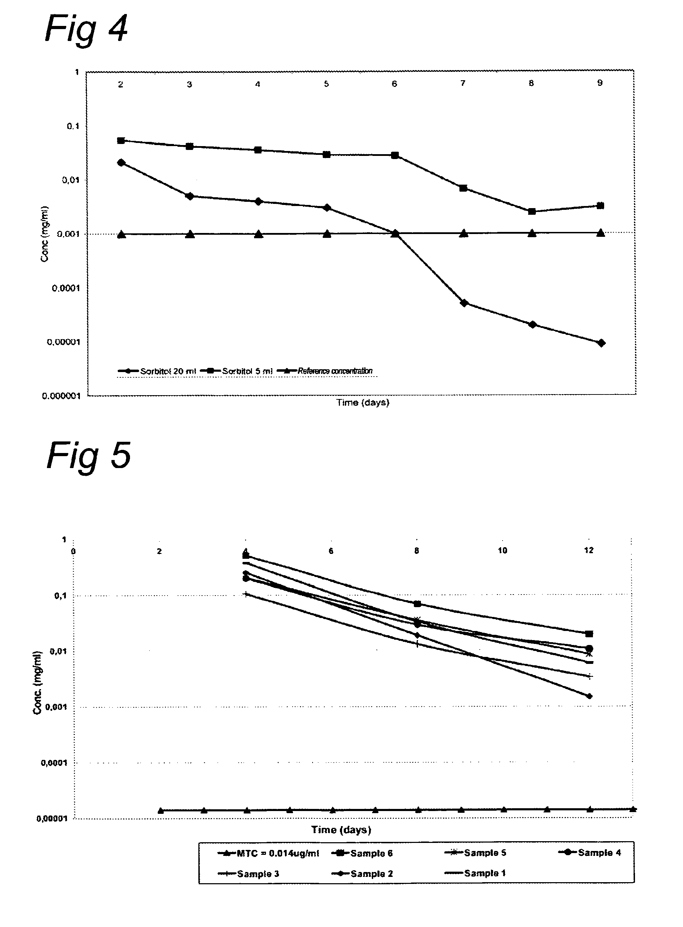

The results so obtained are depicted in Fig. 4. These results indicate that the flow of the release medium had an effect on dexamethasone release from the device. At a flow of 20 ml/day, the reference concentration was reached at t = 6 days, whereas at a flow of 5 ml/day the threshold was not met at t = 9 days.

### Example 9

In example 8, dexamethasone was released relatively quickly from the formulation so, to prolong the release, free dexamethasone was substituted with dexamethasone loaded microparticles of a lipid (Compritol® 888). The formulations tested are described in Table 10.

**Table 10**

| **Name** | **Composition** | **SBF** |
|---|---|---|
| Sample 1 | 9 % dexamethasone, Compritol 888 and 1% Tween. Microspheres made in water | 12.5 ml/day |
| Sample 2 | 9 % dexamethasone, Compritol 888 and 2% Tween. Microspheres made in water | 12.5 ml/day |
| Sample 3 | 9 % dexamethasone, Compritol 888. Melt& mill procedure | 12.5 ml/day |
| Sample 4 | 16 % dexamethasone, Compritol 888. Melt& mill procedure | 12.5 ml/day |
| Sample 5 | 23 % dexamethasone, Compritol 888. Melt& mill procedure | 12.5 ml/day |
| Sample 6 | 33 % dexamethasone, Compritol 888. Melt& mill procedure | 12.5 ml/day |

The release was measured by suspending the plug, formed from the dual composition, in SBF and sampling the medium, which was refreshed at 24 hourly intervals. As a simulation of release, a washout flow of 12.5 ml/24 hours was used. The release medium was analysed by HPLC using UV detection at 246 nm.

The results obtained are depicted in Fig. 5. These results show that loading of lipid microparticles with dexamethasone via the melt/mill procedure afforded a more gradual release profile. The microspheres produced in water with comparable loads afforded a relatively rapid release of dexamethasone; this can be attributed to the smaller particle size of the spheres. Similarly, microspheres produced with a higher surfactant concentration (2 %) were smaller than those produced with 1 % surfactant and had proportionately faster release profiles.

### Example 10

In this example, the dual formulation acted as a vehicle for the sustained delivery of proteins; in this instance, the proteins were albumin and insulin. The dual formulation was prepared as per the methodology described in example 1, with the exception of the addition of the albumin and insulin. The proteins were weighed accurately into a vessel and the sodium alginate / calcium carbonate mixture was added to make up a protein concentration of 1 mg / ml w/v. After thorough mixing of the components, the dual syringe was filled and assembled as described in previous examples. Release of the proteins from the dual formulation after extrusion was measured over time. A fleece "plug", formed from the dual composition, was suspended in simulated body fluid (SBF) and refreshed at 24 hourly intervals. A release medium flow of 20 ml/24 hours was used. The concentration of the proteins in release medium was measured by way of a quantification of protein assay a BCA protein assay kit; using pure albumin proteins as the standards.

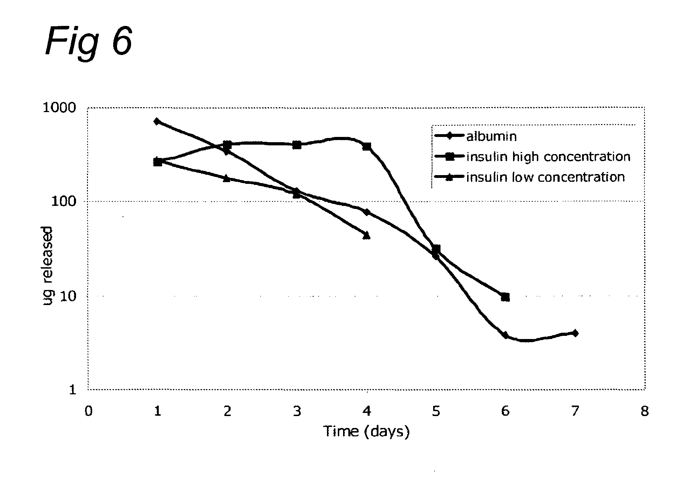

The results so obtained are depicted in Fig. 6. These results indicate that the delivery system has sustained release properties. The proteins used in this example were model compounds; for specific proteins and specific therapeutic aims it will be necessary to adjust the delivery system to get the appropriate release profile. To this aim; techniques that are generally known to specialists in the field of sustained release can be used. For example: manipulation of pore size tuning by using polymers with specific molecular weights; mixtures of several polymers of different molecular weights; adjusting the degree of ionic cross-linking; using different types of cross-linker; combining other hydrogel forming polymers; chemical modification of alginate by the addition of hydrophilic or lipophilic groups etc.

### Example 11

In this example gentamicin alginate beads were prepared and used in a dispensing system according to the present invention.

The gentamicin alginate beads were formed by precipitating an insoluble cross-linked complex from suspension. 50 ml 0.5 - 4 % w/w alginate solution was stirred vigorously while getamicin sulphate solution (1.0 ml, 20 - 50 % w/w in water for injection) was added dropwise. Upon completion of the addition of the gentamicin sulphate solution, the mixture was stirred gently for 15 minutes. The precipitate was hardened by the addition of small amounts of calcium chloride solution and the mixture was stirred for a further 15 minutes. The precipitate was then allowed to sediment after which the supernatant was decanted leaving approximately 10 ml in the vessel. 40 ml absolute ethanol was added and the mixture turraxed to extract water from the sediment. The insoluble particles were allowed to sediment after which the aqueous ethanol was decanted. Residual ethanol was evaporated over a water bath to afford a white powder. The compositions of the first and second flowable composition are depicted in Table 11.

**Table 11**

| **Formulation J** | | | |
|---|---|---|---|
| Reservoir 1 | | Reservoir 2 | |
| Sodium Alginate 3 % w/w | 10 ml | Glycerol | 10 ml |
| | | Gentamicin alginate | 300 mg |
| | | Glucono delta lactone 5 g / 5 ml | 100 mg |

The first dual syringe reservoir was filled with a 3 % solution of sodium alginate, prepared as per the methodology in example 1.

The composition of the second reservoir was prepared as follows. Into a container with 100 mg glucono delta lactone in aqueous medium, gentamicin sulphate (300 mg) was accurately weighed. 10 ml 100 % glycerol was added and the mixture was stirred vigorously for 1 minute. The second reservoir was then filled with the suspension.

The fleece, which forms on mixing of the two components, sets within 1 hour to a soft but solid consistency. A modification to this system may be made for reasons of product stability wherein the gentamicin alginate beads are suspended in the soluble alginate composition.

## Claims

1. A system for delivering a gel-forming fluid, comprising:
• a first reservoir comprising a first flowable composition in the form of an aqueous solution of ionically cross-linkable alginate, said solution containing 0.3-7 wt.% of the alginate;
• a second reservoir comprising a second flowable composition, said second flowable composition containing 30-100 wt.% of one or more polyols; and optionally up to 50 wt.% of water;
• a first outlet from the first reservoir;
• a second outlet from the second reservoir,
• said first outlet and said second outlet being arranged in such a way that the first flowable composition is mixed with the second flowable composition when both compositions are simultaneously expelled from the respective reservoirs, thereby forming the gel-forming fluid;
wherein (i) the first and/or second flowable composition contains 0.1-5 wt.% of an inactive cross-linking agent and the second flowable composition contains 0.01-5 wt.% of an activator or (ii) the second flowable composition contains 0.5-50 wt.% of an active cross-linking agent; and
wherein upon admixture of the first flowable composition with the second flowable composition the cross-linking agent initiates the formation of a solid or semi-solid ionically cross-linked alginate gel.

2. System according to claim 1, wherein the inactive cross-linking agent is a water-insoluble salt of a multivalent cation.

3. System according to claim 2, wherein the inactive cross-linking agent is a water-insoluble salt of a multivalent cation selected from the group consisting of Ca²⁺, Mg²⁺, Zn²⁺, Ba²⁺ and combinations thereof.

4. System according to any one of the preceding claims, wherein the activator is an acidulant.

5. System according to claim 4, wherein the acidulant is selected from the group consisting of lactic acid, tartaric acid, glucono delta lactone, gluconic acid, malic acid, citric acid and combinations thereof.

6. System according to any one of the preceding claims, wherein the one or more polyols are selected from the group consisting of glycerol, sorbitol, mannitol, xylitol and combinations thereof

7. System according to any one of the preceding claims, wherein the second flowable composition contains 0-50 wt.% of water, preferably 0-30 wt.% of water.

8. System according to any one of the preceding claims wherein the system contains the first flowable composition and the second flowable composition in a weight ratio that is within the range 5:1 to 1:5.

9. System according to any one of the preceding claims, wherein the first flowable composition contains 0.1-5 wt.% of the inactive cross-linking agent and has a pH in the range of 7.0-10.5 and the second flowable composition contains 0.01-5 wt.% of the activator and has pH in the range of 1.5-5.

10. System according to claim 10 wherein the polyol is sorbitol and the inactive cross-linking agent is calcium carbonate.

11. System according to any one of claims 1-9, wherein the second flowable composition contains 0.1-5 wt.% of the inactive cross-linking agent and 0.01-5 wt.% of the activator, said second flowable composition having a pH in the range of 1.5-5.

12. System according to claim 12, wherein the polyol is glycerol and the inactive cross-linking agent is gentamicin alginate.

13. System according to any one of claims 1-9, wherein the second flowable composition contains 0.5-50 wt.% of an active cross-linking agent said, said second flowable composition having a pH in the range of 6.5-7.5.

14. System according to claim 14, wherein the polyol is glycerol and the active cross-linking agent is calcium phosphate.

15. System according to any one of the preceding claims, wherein the system is a syringe, said syringe comprising a static mixing unit that is connected to the first outlet of the first reservoir and the second outlet of the second reservoir and which static mixing unit comprises an outlet for dispensing the mixture of the first flowable composition and the second flowable composition.

16. A method of preparing a gel-forming fluid, said method comprising:
• mixing a first flowable composition as defined in claim 1 with a second flowable composition as defined in claim 1; and
• allowing the gel-forming fluid so obtained to form a gel.

## Patentansprüche

1. System zur Abgabe eines gelbildenden Fluids umfassend:
• ein erstes Reservoir umfassend eine erste fließfähige Zusammensetzung in Form einer wässrigen Lösung von ionisch quervernetzbarem Alginat, wobei die Lösung 0,3-7 Gew.-% des Alginats enthält;
• ein zweites Reservoir umfassend eine zweite fließfähige Zusammensetzung, wobei die zweite fließfähige Zusammensetzung 30-100 Gew.-% eines oder mehrerer Polyole enthält; und gewünschtenfalls bis zu 50 Gew.-% Wasser;
• einen ersten Auslass aus dem ersten Reservoir;
• einen zweiten Auslass aus dem zweiten Reservoir,
• wobei der erste Auslass und der zweite Auslass so angeordnet sind, dass die erste fließfähige Zusammensetzung mit der zweiten fließfähigen Zusammensetzung gemischt wird, wenn beide Zusammensetzungen simultan aus den jeweiligen Reservoiren ausgestoßen werden, wodurch das gelbildende Fluid gebildet wird;
wobei (i) die erste und/oder zweite fließ fähige Zusammensetzung 0,1-5 Gew.-% eines inaktiven quervernetzenden Agens enthält und die zweite fließ fähige Zusammensetzung 0,01-5 Gew.-% eines Aktivators enthält oder (ii) die zweite fließfähige Zusammensetzung 0,5-50 Gew.-% eines aktiven quervernetzenden Agens enthält; und
wobei das quervernetzende Agens beim Zusammenmischen der ersten fließ fähigen Zusammensetzung mit der zweiten fließ fähigen Zusammensetzung die Bildung eines festen oder halb-festen ionisch quervernetzten Alginatgels initiiert.

2. System nach Anspruch 1, worin das inaktive quervernetzende Agens ein wasserunlösliches Salz eines multivalenten Kations ist.

3. System nach Anspruch 2, worin das inaktive quervernetzende Agens ein wasserunlösliches Salz eines multivalenten Kations ist, ausgewählt aus der Gruppe bestehend aus Ca2+, Mg2+, Zn2+, Ba2+ und Kombinationen davon.

4. System nach einem der vorhergehenden Ansprüche, worin der Aktivator ein Ansäuerungsmittel ist.

5. System nach Anspruch 4, worin das Ansäuerungsmittel ausgewählt ist aus der Gruppe bestehend aus Milchsäure, Weinsäure, Glukono-delta-lacton, Glukonsäure, Äpfelsäure, Zitronensäure und Kombinationen davon.

6. System nach einem der vorhergehenden Ansprüche, worin das eine oder mehrere Polyole ausgewählt sind aus der Gruppe bestehend aus Glycerol, Sorbitol, Mannitol, Xylitol und Kombinationen davon.

7. System nach einem der vorhergehenden Ansprüche, worin die zweite fließfähige Zusammensetzung 0-50 Gew.-% Wasser enthält, vorzugsweise 0-30 Gew.-% Wasser.

8. System nach einem der vorhergehenden Ansprüche, worin das System die erste fließfähige Zusammensetzung und die zweite fließfähige Zusammensetzung in einem Gewichtsverhältnis enthält, das innerhalb eines Bereichs von 5:1 bis 1:5 liegt.

9. System nach einem der vorhergehenden Ansprüche, worin die erste fließfähige Zusammensetzung 0,1-5 Gew.-% des inaktiven quervernetzenden Agens enthält und einen pH im Bereich von 7,0-10,5 besitzt und die zweite fließfähige Zusammensetzung 0,01-5 Gew.-% des Aktivators enthält und einen pH im Bereich von 1,5-5 besitzt.

10. System nach Anspruch 10, worin das Polyol Sorbitol ist und das inaktive quervernetzende Agens Calciumcarbonat ist.

11. System nach einem der Ansprüche 1-9, worin die zweite fließfähige Zusammensetzung 0,1-5 Gew.-% des inaktiven quervernetzenden Agens und 0,01-5 Gew.-% des Aktivators enthält, wobei besagte zweite fließfähige Zusammensetzung einen pH im Bereich von 1,5-5 besitzt.

12. System nach Anspruch 12, worin das Polyol Glycerol ist und das inaktive quervernetzende Agens Gentamicinalginat ist.

13. System nach einem der Ansprüche 1-9, worin die zweite fließfähige Zusammensetzung 0,5-50 Gew.-% eines genannten aktiven quervernetzenden Agens enthält, wobei die zweite fließfähige Zusammensetzung einen pH im Bereich von 6,5-7,5 besitzt.

14. System nach Anspruch 14, worin das Polyol Glycerol ist und das aktive quervernetzende Agens Calciumphosphat ist.

15. System nach einem der vorhergehenden Ansprüche, worin das System eine Spritze ist, wobei die Spritze eine statische Mischeinheit umfasst, die an den ersten Auslass des ersten Reservoirs und den zweiten Auslass des zweiten Reservoirs angeschlossen ist und die statische Mischeinheit einen Auslass zur Abgabe der Mischung der ersten fließfähigen Zusammensetzung und der zweiten fließfähigen Zusammensetzung umfasst.

16. Verfahren zum Herstellen eines gelbildenden Fluids, wobei das besagte Verfahren umfasst:
• Mischen einer ersten, wie in Anspruch 1 definierten fließfähigen Zusammensetzung mit einer zweiten, wie in Anspruch 1 definierten fließfähigen Zusammensetzung; und
• Ermöglichen für das so erhaltene gelbildende Fluid, ein Gel zu bilden.

## Revendications

1. Système de délivrance d'un fluide gélifiant, comprenant :
- un premier réservoir comprenant une première composition fluide sous la forme d'une solution aqueuse d'alginate ioniquement réticulable, ladite solution contenant de 0,3 à 7 % en poids de l'alginate ;
- un second réservoir contenant une seconde composition fluide, ladite seconde composition fluide contenant de 30 à 100 % en poids d'un ou plusieurs polyols ; et facultativement jusqu'à 50 % en poids d'eau ;
- un premier orifice de sortie du premier réservoir ;
- un second orifice de sortie du second réservoir,
- ledit premier orifice de sortie et ledit second orifice de sortie étant configurés de telle manière que la première composition fluide est mélangée à la seconde composition fluide lorsque les deux compositions sont simultanément expulsées des réservoirs respectifs, formant ainsi le fluide gélifiant ;
dans lequel (i) la première composition fluide et/ou la seconde composition fluide contiennent de 0,1 à 5 % en poids d'un agent de réticulation inactif et la seconde composition fluide contient de 0,01 à 5 % en poids d'un activateur ou (ii) la seconde composition fluide contient de 0,5 à 50 % en poids d'un agent de réticulation actif ; et
dans lequel sous l'effet du mélange de la première composition fluide avec la seconde composition fluide l'agent de réticulation initie la formation d'un gel d'alginate ioniquement réticulé solide ou semi-solide.

2. Système selon la revendication 1, dans lequel l'agent de réticulation inactif est un sel insoluble dans l'eau d'un cation multivalent.

3. Système selon la revendication 2, dans lequel l'agent de réticulation inactif est un sel insoluble dans l'eau d'un cation multivalent sélectionné dans le groupe constitué du C²⁺, du M²⁺, du Zn²⁺, du Ba²⁺ et de leurs combinaisons.

4. Système selon l'une quelconque des revendications précédentes, dans lequel l'activateur est un acidulant.

5. Système selon la revendication 4, dans lequel l'acidulant est sélectionné dans le groupe constitué de l'acide lactique, de l'acide tartrique, de la glucono-delta-lactone, de l'acide gluconique, de l'acide malique, de l'acide citrique et de leurs combinaisons.

6. Système selon l'une quelconque des revendications précédentes, dans lequel le ou les polyols sont sélectionnés dans le groupe constitué du glycérol, du sorbitol, du mannitol, du xylitol et de leurs combinaisons.

7. Système selon l'une quelconque des revendications précédentes, dans lequel la seconde composition fluide contient de 0 à 50 % en poids d'eau, de préférence de 0 à 30 % en poids d'eau.

8. Système selon l'une quelconque des revendications précédentes, dans lequel le système contient la première composition fluide et la seconde composition fluide en un rapport en poids qui se trouve dans la plage de 5/1 à 1/5.

9. Système selon l'une quelconque des revendications précédentes, dans lequel la première composition fluide contient de 0,1 à 5 % en poids de l'agent de réticulation inactif et a un pH dans la plage de 7,0 à 10,5 et la seconde composition fluide contient de 0,01 à 5 % en poids de l'activateur et a un pH dans la plage de 1,5 à 5.

10. Système selon la revendication 10, dans lequel le polyol est le sorbitol et l'agent de réticulation inactif est le carbonate de calcium.

11. Système selon l'une quelconque des revendications 1 à 9, dans lequel la seconde composition fluide contient de 0,1 à 5 % en poids de l'agent de réticulation inactif et de 0,01 à 5 % en poids de l'activateur, ladite seconde composition fluide ayant un pH dans la plage de 1,5 à 5.

12. Système selon la revendication 12, dans lequel le polyol est le glycérol et l'agent de réticulation inactif est l'alginate de gentamicine.

13. Système selon l'une quelconque des revendications 1 à 9, dans lequel la seconde composition fluide contient de 0,5 à 50 % en poids d'un agent de réticulation actif, ladite seconde composition fluide ayant un pH dans la plage de 6,5 à 7,5.

14. Système selon la revendication 14, dans lequel le polyol est le glycérol et l'agent de réticulation actif est le phosphate de calcium.

15. Système selon l'une quelconque des revendications précédentes, dans lequel le système est une seringue, ladite seringue comprenant une unité de mélange statique qui est raccordée au premier orifice de sortie du premier réservoir et au second orifice de sortie du second réservoir et laquelle unité de mélange statique comprend un orifice de sortie pour distribuer le mélange de la première composition fluide et de la seconde composition fluide.

16. Procédé de préparation d'un fluide gélifiant, ledit procédé comprenant :
- le mélange d'une première composition fluide selon la revendication 1 avec une seconde composition fluide selon la revendication 1 ; et
- le fait de laisser le fluide gélifiant ainsi obtenu former un gel.
